# NEW EUROPEAN PATENT SPECIFICATION

(11) **EP 1 172 041 B2**
(45) Date of publication and mention of the opposition decision: **02.09.2009**
(45) Mention of the grant of the patent: 07.12.2005
(21) Application number: 01123879.7
(22) Date of filing: 04.08.1997
(51) Int. Cl.: A23L 1/09, A23L 1/0526, A23L 1/053, A23L 1/0534, A23L 1/054, A23L 1/0524, A23L 1/0528, A23L 1/0532, A23L 1/212, A23L 1/30

(54) **Compositions of plant carbohydrates as dietary supplements**
Zusammensetzungen auf pflanzlicher Kohlenhydratbasis als Nahrungsergänzungsstoffe
Compléments nutritionnels à base d'hydrates de carbone végétaux

(30) Priority: 09.08.1996 US 22467 P; 01.11.1996 US 30317 P; 24.07.1997 US 57017 P
(43) Date of publication of application: 16.01.2002
(62) Divisional of application: 97935205.1
(73) Proprietor: Mannatech, Inc., Coppell, TX 75019 (US)
(72) Inventor: McAnalley, Bill H., Grand Prairie, Texas 75052 (US); Moore, Eric D., Grapevine, Texas 76057 (US); Fioretti, William C., Grapevine, Texas 76051 (US); McDaniel, Reginald H., Mansfield, Texas 76063 (US); Vennum, Eileen P., Grand Prairie, Texas 75050 (US)
(74) Representative: Hanna, Peter William Derek

(56) References cited:
- EP-A- 0 521 707
- DE-A- 3 935 906
- DATABASE WPI Section Ch, Week 198324 Derwent Publications Ltd., London, GB; Class D13, AN 1983-57457K XP002201718 & JP 58 076065 A (KAGAWA Y), 9 May 1983 (1983-05-09)
- PATENT ABSTRACTS OF JAPAN vol. 016, no. 054 (C-0909), 12 February 1992 (1992-02-12) & JP 03 255029 A (NISSHIN FLOUR MILLING CO LTD), 13 November 1991 (1991-11-13)
- PATENT ABSTRACTS OF JAPAN vol. 1996, no. 01, 31 January 1996 (1996-01-31) -& JP 07 242551 A (FUJISAWA PHARMACEUT CO LTD), 19 September 1995 (1995-09-19)
- DANIEL BEN-GHEDALIA AND JOSHUA MIRON: 'The Digestion of Total and Cell Wall Monosaccharides of Alfalfa by Sheep' JOURNAL OF NUTRITION vol. 114, 1984, pages 880 - 887
- ROBERT K. MURRAY, PETER A. MAYES ET AL.: 'Harper's Biochemistry', vol. 24, March 1996, APPLETON & LANGE, CONNECTICUT article ROBERT K. MURRAY: 'Glycoproteins', pages 648 - 666

## Description

### FIELD OF THE INVENTION

This invention pertains to the field of dietary supplements and nutritional support for promotion and maintenance of good health. More specifically, the invention relates to compositions of carbohydrates as dietary supplements that are essential for the production of correctly structured and, therefore, properly functioning glycoproteins.

### DESCRIPTION OF THE PRIOR ART AND OTHER INFORMATION

The term mucus was first used in the 1700s. By 1805, Bostok realized that mucus was composed of protein that differed from albumin and gelatin. In 1865, Eichwald showed that mucins contained carbohydrate moieties. In 1877, Hoppe-Seyler discovered that mucins were high in sialic acid content. In 1882, Landwehr showed that plant gums, a type of mucin, contain more than one monosaccharide. With the advent of more modern methods, these monosaccharides were isolated and characterized. In 1888, Harmarsten showed that the saccharides in mucins were joined by a covalent bond; Harmarsten was the first to use the term "glykoproteide" (or glycoprotein in English). Fischer and Leuchs discovered high concentrations of mannose in mucus in 1902. Hayworth, in 1939, discovered N-acetylglucosamine and Bierry discovered galactose in 1930. Meyer discovered fucose in 1958 (Gottschalk, Glycoproteins, 1972).

Proteins were originally thought to be the primary "communication" molecules of the body. The biotechnology revolution began as an attempt to create new drugs based upon proteins which are made up of various combinations of amino acids. However, since amino acids can only bind to each other through an amide bond, the number of secondary configurations possible with proteins is limited. Indeed, only one secondary configuration is possible per dipeptide.

However, many more functions are performed by the body than can be accounted for by the number of molecular configurations possible with proteins. Several years ago a theoretical mathematician calculated the number of configurations possible with proteins and discovered that another mechanism, yet unknown, had to be responsible for performing most of the communication functions of the body. It is now known that this mechanism involves carbohydrates.

In contrast to the simpler proteins, more molecular configurations are possible with the more complex carbohydrate molecule, e.g., a hexose has six chiral centers each of which has two isomeric forms and each of which has a hydroxyl group as a binding site for other molecules. Thus, while only 24 oligopeptide configurations are possible with four amino acids, more than 100,000 different oligosaccharide configurations are possible with four sugars (Stryer et al., Biochemistry, 1995; p 477),

Science has recently shown that glycoproteins play a key role in all cellular communication. Many of the cytokines, i.e. cellular messenger agents, do not function properly without an attached glycosyl moiety. The body hydrolyzes complex polysaccharides such as plant carbohydrates into various monosugars and restructures them into oligosaccharides that are then used by the body to build the glycoproteins required by cytokines for cellular communication and, thus, for good health.

With the advent of improved analytical techniques and more powerful computers, characterization of glycoproteins increased rapidly after the 1960s. By the mid 1980s, the mechanism of the orderly synthesis of glycoproteins in the endoplasmic reticulum and Golgi apparatus had been determined. The actual oligosaccharide conformations of many glycoproteins are now known.

Increasing interest in glycobiology has been precipitated by recent findings that cell surface carbohydrates are critically involved in cell adhesion and, thus, in cell-cell interaction. Specifically, three new mechanistic concepts have been discovered. First, structural studies in glycoproteins and glycolipids have revealed the existence of carbohydrates which are unique to certain cell types. This concept is crucial to understanding cell surface carbohydrates as cell-type specific recognition molecules.

A second concept was developed from new information regarding lectins, which have sugar-binding proteins. In the 1970s it was learned that glycoproteins were removed rapidly from the blood when their sialic acid, i.e. N-acetylneuraminic acid, containing branches were removed. Further studies revealed that this rapid clearance was caused by asialoglycoproteins binding to lectins that recognize terminal galactose. Once animal cells were known to have lectins, a large number of lectins were characterized, and a dedicated section in the amino acid sequence that is responsible for the carbohydrate recognition domain in the lectins was discovered. This discovery was critical to understanding carbohydrate-binding capability in cell-cell interactions. Thus, cellular communication was recognized at the molecular level.

The third concept resulted from studies regarding the isolation and characterization of the glycosyltransferases that form carbohydrates. These studies showed that carbohydrate moieties are usually built one by one, and each reaction is carried out by a glycosyltransferase that forms only a specific linkage. The advent of molecular biology in this field has enabled scientists to manipulate carbohydrate expression and study glycoprotein function.

Based on critical advances in this field, the most recent studies demonstrated that oligosaccharides uniquely present in leukocytes act as ligands for adhesive molecules in endothelia and platelets. When these adhesive molecules, known as selectins, were cloned, it was discovered that they contained carbohydrate recognition domains. Thus, studies on cell-type specific carbohydrates and animal lectins corroborated each other. Moreover, these studies were preceded by the findings that lymphocyte-endothelial interaction is dependent upon carbohydrates.

Given the above, research directed toward the synthesis of drugs that would correct malformation of glycoproteins on cell surfaces began. After the carbohydrate ligand sialyl-Le' was identified, pharmaceutical companies soon synthesized it for therapeutic purposes. This line of research has since become much easier because enzymatic synthesis of carbohydrates is now possible thanks to the availability of glycosyltransferases generated by cloned cDNAs (Fukuda et al., Glycobiology, 1994).

The synthesis of all proteins and glycoproteins is controlled by somatic genes embodied in the chromosomes of a cell. The coding information expressed in nucleic (DNA) controls all cellular functions, including general body defense, regeneration, remodeling and healing. Though DNA provides the blueprint, the cellular components cannot be built correctly without the required building blocks. As discussed above, cytokines are key components used for intracellular instruction to carry out the body's vital functions. However, many cytokines do not function properly without an attached glycosyl moiety.

Table 1 lists some of the known physiological functions served by glycoproteins. Table 2 lists some of the specific known functions that the oligosaccharide branches or chains of glycoproteins perform.

**Table 1.**

| **Some known functions served by glycoproteins:** | |
|---|---|
| Function | Glycoproteins |
| Structural molecule | Collagens |
| Lubricant and protective agent | Mucins |
| Transport molecule | Transferrin, ceruloplasmin |
| Immunologic molecule | Immunoglobulins, histocompatibility antigens |
| Hormone | Chorionic gonadotropin, thyroid-stimulating hormone (TSH) |
| Enzyme | Various, e.g., alkaline phosphatase |
| Cell attachment-recognition site | Various proteins involved in cell-cell (e.g., sperm-oocyte), virus-cell, bacterium- cell, and hormone-cell interactions |
| Interact with specific carbohydrates | Some lectins |

**Table 2.**

| **Some known functions of the oligosaccharide chains of glycoproteins:** |
|---|
| * Modulate physicochemical properties, e.g., solubility, viscosity, charge, and protein denaruration |
| * Protect against proteolysis from within and outside the cell |
| * Affect proteolytic processing of precursor proteins to smaller products |
| * Are involved in biologic activity, e.g., of human chorionic gonadotropin (hCG) |
| * Affect insertion of protein into membranes, intracellular protein migration, and protein sorting and secretion |
| * Affect embryonic development and differentiation |
| * Affect metabolism |
| * May affect sites of metastases selected by cancer cells |

In summary, various processes of the cell are regulated or affected by correctly structured and, therefore, properly functioning glycoproteins.

Despite the above discussed current scientific knowledge concerning the importance of to cell-cell communication and the importance of carbohydrates in the formation of glycoproteins, and despite the fact that diet is the source of a majority of carbohydrates, the fields of glycobiology and nutrition have never been adequately investigated together. Although current nutrition textbooks stress the importance of essential vitamins, minerals, proteins (amino acids) and fats in great detail, sugars are currently recognized only as a source of energy (Shils et al., 1994)- not as substances essential to glycoprotein production for good health. For example, Shils et al. disclose that the principal sources of dietary carbohydrates are: 1) maize, rice, wheat, and potato which yield starches comprising glucose; 2) sugar cane and beet sugar which yield fructose and glucose; and 3) milk which yields galactose and glucose (Shils et al., Modern Nutrition in Health and Disease, (1994)).

By way of contrast, Harper's Biochemistry (Murray et al., 1995) lists eight and Principles of Biochemistry, Vol II (Zubay et al., 1995) lists eleven monosaccharides commonly found in the oligosaccharide chains of cellular glycoproteins. Thus, of the approximate 200 monosaccharides found in nature, these eleven are believed to be important toward maintaining good health in mammals.

These eleven saccharides include galactose, glucose, mannose, N-acetylneuraminic acid, fucose, N-acetylgalactosamine, N-acetylglucosamine and xylose (Murray et al., Harper's Biochemistry, 1996) as well as iduronic acid, arabinose and glucuronic acid, (Zubay et al., Principles of Biochemistry, Vol II, 1995). The structures of these carbohydrates are disclosed in Stryer's Biochemistry (Stryer, 1995) and the Merck Index, 12th Edition, 1996.

Recognizing this, scientists are currently trying, as yet with limited success, to synthetically attach glycosyl moieties to cytokines and other proteins. In fact, NIH has launched a project to develop methods to synthesize the glyco portion currently missing from their genetically engineered proteins. These synthetically produced cytokines have so far demonstrated disappointing results. Many challenges remain in this area. Scientists must first learn: 1) how to synthesize the glyco portion, 2) how to attach the glyco portion to the protein, and then 3) how to get the correct glycoproteins in the right concentrations to the right places in the body so as to facilitate good health.

For centuries, people of diverse cultures from around the world have utilized plants and herbs in the treatment of a wide variety of disorders in mammals. Specifically, formulations including poultices, teas, powders, pastes, extracts, plant or herb parts, plant or herbal extracts, lotions, creams, salves, troches, and others have been used. It is also now well recognized that much of the world's farm lands have been depleted of essential minerals required to sustain life, thus requiring the widespread use of vitamin, mineral and dietary supplements. A recent discovery concerns the importance of plant chemicals (phytochemicals) that are found in vine-ripened fruits and vegetables but are not found in those that are not vine-ripened. To provide these necessary, yet undefined, phytonutrients or phytonutritionals, as defined below, to the diet, some companies have begun supplying dietary supplements of freeze-dried, vine-ripened fruits and vegetables.

Nutritionists have developed hundreds of dietary supplement formulations in an effort to provide essential dietary components and facilitate and promote good health in mammals. However, fraudulent product claims regarding the treatment of physiological disorders are pervasive in the industry, and modern farming methods which focus on volume rather than nutritional value of crop production have led to crops having reduced dietary value that are missing essential dietary components.

Despite the extremely large number of dietary supplements available on store shelves today, the dietary needs of humans are still not being met. Many of such commercially available dietary supplements do not appear to provide any significant nutritional benefit. The present inventors believe such prior products suffer any one or more of the following disadvantages; a) they do not include the correct nutritional product(s); and b) their nutritional products are not well absorbed by a person taking them.

Thus, while scientists are beginning to recognize that other phytochemicals are required for good health, and others have previously recognized the utility of plants and herbs in the treatment of disorders, none of the known art suggests or discloses the invention as claimed herein. A need remains for non-pharmaceutical based dietary supplement formulations which provide essential saccharides that are the building blocks of glycoproteins and which promote good health in mammals.

DE-A-3,935,906 describes a nutritional composition to improve the anabolism of patients with metabolic deficits. The composition comprises at least 5 wt. % of the monosaccharide fraction consisting of compounds selected from the group;
D-galactose, L-fucose, D-mannose, D-glucosamine, N-acetyl-D-galactosamine, N-acetylmannosamine, D-lactose and D-lactulose, alone or in mixtures thereof in which 50 mol % consists of D-galactose, its derivatives and precursors.

### SUMMARY OF THE INVENTION

It is an object of the present invention to provide a dietary supplement which promotes good health by providing to a mammal essential saccharides which are the building blocks of glycoproteins.

It has now been demonstrated herein by the present inventors that inclusion of these essential saccharides, as by supplementation of a diet with a dietary supplement containing the same, in the diets of mammals, promotes good health. Although not intended to be limited to a particular mechanism of action, these essential saccharides are believed to be absorbed into the mammal's body and utilized in the formation of glycoproteins. By so providing these essential saccharides, the mammal's body does not have to spend energy unnecessarily catabolizing these essential saccharides and can therefore spend its energy providing for other physiological needs such as enhancement of the immune system to ward off, combat and/or ameliorate a wide range of physiological disorders.

Thus, the present invention overcomes the disadvantages and drawbacks of the prior art. One aspect of the present invention is directed to the use of various compositions of carbohydrates, i.e., glyconutritionals or glyconutrients, as dietary supplements which supplement a mammal's diet with sugars essential to glycoprotein and/or glycolipid production and thereby promote good health.

The present invention provides a bioabsorbable dosage form for providing nutritional product saccharides, in accordance with the claims which follow.

### DETAILED DESCRIPTION

The body of a mammal hydrolyzes or metabolizes complex polysaccharides, such as plant carbohydrates, into various monosaccharides and subsequently forms oligosaccharides therefrom that are then used by the body to build the glycoproteins required by cytokines for cellular communication.

As used herein the term "phytochemical" refers to plant synthesized molecules, found in food, or plant tissue in a complex organic matrix, which are minimally altered by processing from how they occur in nature. As used herein, the term "nutraceutical" refers to a non-toxic, nutrient of plant, mineral or animal origin, that has health promoting activity and that can be standardized and supplied as a dietary supplement to improve the nutritional quality of a balanced general diet. A nutraceutical is also a glyconutrient or phytonutrient.

As used herein, the terms "glyconutritional" or "glyconutrient" refer to complex carbohydrates or saccharides or simple sugars that are synthesized in nature and are necessary for the biochemical synthesis of various classes of communication and signal molecules that may be free in interstitial cellular fluids, active in cell to cell communication (i.e., cytokines, growth factors, etc.), or constitute the molecular configuration comprising foci of highly specific molecular activity of cell membranes (i.e., receptor sites, ion-transport channels, antigenic identification, and the like).

As used herein, the terms "phytonutritional" or "phytonutrient" refer to naturally synthesized molecules found only in plants that are produced to protect the plant's cells. Phytonutrients primarily have antioxidant, free-radical scavenger and vital micronutrient activity. These molecules, supplied through dietary supplementation, are found in mature plant tissues, and are most concentrated in seed coats and fruiting tissues surrounding the seed. In mammalian tissues, these molecules when supplied in the diet, are active in optimizing the biochemistry, immunology and physiology in the cellular micro-environment.

As used herein, the term "dioscorea complex" refers to an extract of dioscorea species (Mexican yam) providing a natural pre-cursor, dietary nutrient, diosgenin, a complex, six-ring, cyclic-carbon molecule that contains the molecular scaffold (perhydrocyclopentanophenanthrene) upon which mammalian adrenal and gonadal hormones are naturally synthesized. Providing this complex molecule in the diet can support optimal hormone balance, while maintaining normal physiological control mechanisms. This dietary supplement component has the potential to improve metabolic regulation of virtually every functioning cell in the body.

As used herein, the term "herbal extract" refers to phytochemicals that are produced in plant tissues and that can be extracted by water, polar, or petroleum solvents, and that have some degree of beneficial health or therapeutic activity. Most herbal agents can be toxic, especially when concentrated, but are generally safe when utilized in their more traditional manner in teas and poultices as a "folk medicinal for the treatment of disease and promotion of good health. As used herein, the term "herbal body-toning agent" refers to substances that have been observed by the inventors to reduce and reverse elastic tissue and collagen fiber damage caused by aging or sun-damage as evidenced by a restoration of skin turgor and elasticity which effectively reduces or eliminates wrinkles, sagging, hyperpigmentation and reversal of other undesirable elements of lost cosmetic appearance.

The carbohydrates included in the dietary supplement of the invention are available from a wide variety of natural and synthetic sources such as shrubs, trees, plants, yeasts, fungi, molds, gums, resins, starch and cellulose derivatives and natural mucin sources. Specifically, some of the natural sources include: (a) shrub or tree exudates which contain acacia, karaya, tragacanth, or ghatti; (b) marine gums which include agar, algin, or carrageenan; (c) seed gums which include guar, locust bean, or psyllium; (d) plant extracts which contain pectins or acetylated polymannose; (e) starch and cellulose derivatives such as hetastarch, carboxymethylcellulose, ethylcellulose, hydroxypropyl methylcellulose, methylcellulose, oxidized cellulose; and microbial gums which contain dextrans, xanthan. (Tyler et al., 1981). However, it should be recognized that the composition of the invention is not intended to be limited by the source from which the respective carbohydrates are obtained.

The saccharides of the invention can be found in nature as mono-, oligo- and/or polysaccharides. The compositions of the invention contain the saccharides in their monomeric form. Table 3 below lists some of the known natural sources for the saccharides of the invention.

**Table 3.**

| **Natural sources of saccharides.** | |
|---|---|
| Source Carbohydrate | Corresponding Saccharide(s) |
| gum tragacanth | galacturonic acid, galactose, fucose, xylose, arabinose and rhamnose |
| guar gum | mannose and galactose (1:2 molar ratio) |
| rice or grain flour | glucose |
| LAREX B-1000 (Larch tree extract) | polyarabinogalactan |
| MANAPOL™ (aloe vera extract) | acetylated mannose based polymer |
| gum ghatti | arabinose, galactose, mannose, xylose, glucuronic acid (10:6:2:1:2 molar ratio) |
| starch | glucose |
| pectin | galacturonic acid |
| chondroitin sulfate | N-acetylgatactosamine |
| chitin | N-acetylglucosamine |
| acacia, gum arabic | arabinose, galactose, glucuronic acid |
| alginic acid | mannosyluronic acid, gulosyluronic acid |
| carrageenan | galactose, 3,6-anhydrogalactose |
| dextran | glucose |
| xanthan gum | glucose, mannose, glucuronic acid |

It is well recognized in the art that the saccharides listed above with their corresponding source carbohydrates are present in particular amounts in nature as exemplified by the indicated molar ratios for the saccharides in gum ghatti and guar gum. The relative amounts or ratios of saccharides in natural carbohydrates is readily determined using conventional extraction or analytical methods or can be obtained from literature sources commonly used in the art.

As used herein, the term "carbohydrate" is used interchangeably with the terms "saccharide", "polysaccharide", "oligosaccharide" and "sugar" the definitions of which are well known in the art of carbohydrate chemistry. Although the compositions of the invention are intended to include at least one of the eleven essential saccharides, it should be noted that the saccharides selected are in the form of monosaccharides.

Although the present invention includes the above cited eleven essential saccharides, it should be noted that other saccharides, nutritional compounds or biologically active or inert compounds can be included in the dietary supplement of the invention. Such other nutritional compounds include any one or more of phytonutrients, dioscorea complex, plant extracts, herbal extracts, plant parts, herbal components, vitamins or minerals. These nutritional compounds can be added to the dietary supplement of the invention, or they can be provided separately to a mammal being administered said dietary supplement. For example, a person receiving the glyconutrient-containing dosage form of the invention can also receive a phytonutrient in either the same or a separate dosage form. Inert compounds can include flavors, fillers, lubricants, buffers, gels, binders, excipients, carriers and/or other such compounds that facilitate the formulation or administration of the inventive dietary supplement. All of the glyconutrient-containing dietary supplement compositions of the invention, even those containing additional compounds, agents or other substances, can be obtained directly from MANNATECH™ (Coppell, TX).

Dioscorea complex is available from Ayusherbs (Japan). When dioscorea complex is included in the dietary supplement of the invention, the ratio of diascorea complex to total essential saccharide can range from about 0.0001/99.9999 to about 50/50 on a weight percent basis. In particular embodiments, the dioscorea complex to total essential saccharide ratio ranges from about 0.01-70/99.99-30 or about 10-40/90-60 or about 20/80.

Phytonutrients are available from a wide variety of manufacturing sources such as Cap-Tab (U.S.) or they can be added by freeze-drying and grinding ripe fruits and/or vegetables to form a powder which can then be added to or provided along with the dietary supplement of the invention. Such fruits and vegetables can be selected from all known fruits and vegetables but, in particular exemplary embodiments, include broccoli, Brussels sprouts, cabbage, carrot, cauliflower, garlic, kale, onion, papaya, pineapple, tomato and turnip. These phytonutrients can be formulated in powder-containing caplet or capsule forms or in a base of gelatin and natural fruit fructose, optionally containing added flavors. When a phytonutrient is included in the dietary supplement of the invention, the ratio of total phytonutrient to total glyconutrient can range from about 0.001/99.999 to about 99.99/0.01 on a weight percent basis. In particular embodiments, the phytonutrient to total glyconutrient ratio ranges from about 20-99/80-1 or about 50-95/50-5.

There are many plant and herbal ext-acts with suspected or demonstrated nutritional value which can promote good health and can be incorporated in or administered along with the dietary supplement of the invention. Such plant and herbal extracts can be obtained according to well known procedures for the extraction of substances, compounds or agents from plants or herbs. In particular embodiments, the dietary supplement of the present invention includes herbal or plant extracts of broccoli, Brussels sprouts, cabbage, carrot, cauliflower, garlic, kale, onion, papaya, pineapple, tomato, asparagus, mushroom, parsnip, radish, and turnip. When a plant or herbal extract is included in the dietary supplement of the invention, the ratio of total extract (dry solids weight basis) to total glyconutrient can range from about 0.001-75/99.999-25 to about 10-90/90-10 on a weight percent basis.

Many different types of vitamins and minerals can be included in the dietary supplement of the invention. While a few vitamins and minerals of synthetic origin do possess nutritional value, particular embodiments of the dietary supplement herein contain nutritionally effective amounts of non-toxic vitamins and minerals obtained predominantly from natural sources. PROFILE™ is the trade name of a vitamin and mineral supplement used in the nutritional studies exemplified herein. This product, which can be obtained from MANNATECH™ (Coppell, TX), contains nutritionally effective amounts of the following vitamins and minerals: a) vitamins comprising A, B1, B12, B2, B6, beta carotene, bioflavanoids, biotin, C, choline, D, E, folic acid, inositol, K, niacinamide, para-aminobenzoic acid, and pantothenic acid; and b) minerals comprising boron, calcium, copper, GTF chromium, iodine, iron, magnesium, manganese, molybdenum, potassium, selenium, silicon, vanadium, and zinc. These vitamins and minerals may be provided in nutritionally acceptable non-toxic forms.

By "nutritionally effective amount" is meant that amount which will provide a beneficial nutritional effect or response in a mammal. For example, as nutritional response to vitamin- and mineral-containing dietary supplements varies from mammal to mammal, it should be understood that nutritionally effective amounts of said vitamins and minerals will vary, respectively. Thus, while one mammal may require a particular profile of vitamins and minerals present in defined amounts, another mammal may require the same particular profile of vitamins and minerals present in different defined amounts.

Other compounds, agents and nutrients can also be included in the dietary supplement of the invention, such as, for example, cellulose, calcium carbonate, kola nut, kola nut extract, country mallow, Atlantic kelp, cayenne pepper, silica, stearic acid, amino acids, glycine, lysine, glutamic acid, arginine, calcium carbonate, orchic substances, boron citrate, chromium picolinate, essential fibers, essential oils, essential botanicals, essential enteric ecology and flora growth promoters, essential fatty acids, live probiotic flora, proteins and enzymes.

The dietary supplement of the invention has been prepared and administered to mammals in powdered, reconstitutable powder, liquid-solid suspension, liquid, capsule, tablet, caplet, lotion and cream dosage forms. It should be readily obvious to one of ordinary skill in the science of formulations that the present dietary supplement can also be formulated appropriately for irrigation, ophthalmic, otic, rectal, sublingual, transdermal, buccal, vaginal, or dermal administration. Thus, other dosage forms such as chewable candy bar, concentrate, drops, elixir, emulsion, film, gel, granule, chewing gum, jelly, oil, paste, pastille, pellet, shampoo, rinse, soap, sponge, suppository, swab, syrup, chewable gelatin form, or chewable tablet can be used.

Due to varying diets among people, the dietary supplement of the invention can be administered in a wide range of dosages and formulated in a wide range of dosage unit strengths. For example, for those people who are missing from their diet nine of the eleven essential saccharides, a dietary supplement containing those nine saccharides in nutritionally effective amounts can be formulated. As well, for those people whose bioabsorption of essential saccharides is extremely efficient, a dietary supplement formulation containing reduced amounts of essential saccharides can be prepared.

It should be noted that the dosage of the dietary supplement can also vary according to a particular ailment or disorder that a mammal is suffering from when taking the supplement. For example, a person suffering from chronic fatigue syndrome or fibromyalgia, will generally require a dose different from an alcoholic who is trying to discontinue alcohol consumption in order to obtain a nutritional benefit. An appropriate dose of the dietary supplement can be readily determined by monitoring patient response, i.e., general health, to particular doses of the supplement. As well, when another agent such as a phytonutrient, plant extract, herbal extract and/or dioscorea complex is being administered to a mammal along with the present glyconutritional dietary supplement, the appropriate doses of the supplement and each of the agents can be readily determined in a like fashion by monitoring patient response, i.e. general health, to particular doses of each.

It is contemplated by the invention that the dietary supplement can be administered simultaneously or sequentially in one or a combination of dosage forms, While it is possible and even likely that the present dietary supplement will provide an immediate overall health benefit, such benefit may take days, weeks or months to materialize. Nonetheless, the present glyconutritional dietary supplement will provide a beneficial nutritional response in a mammal consuming it.

It is also contemplated that the dietary supplement of the invention can be administered simultaneously or sequentially along with at least one of a phytonutrient, an herbal extract, a plant extract, and a dioscorea complex. Particular embodiments wherein the dietary supplement is administered simultaneously with at least one of a phytonutrient, an herbal extract, a plant extract, and a dioscorea complex are exemplified in the following examples.

For example, the dietary supplement of the invention was administered as a powder-containing capsule. When the dietary supplement included a phytonutrient, it was administered as a caplet or gelatin form. When the dietary supplement included a diascorea complex, it was administered as either a capsule or caplet. When the dietary supplement included a phytonutrient, a dioscorea complex and an herbal extract, it was administered as a caplet.

According to the capsule or caplet size and ingredients used in a study, the dietary supplement was administered initially as follows. The indicated doses are based upon #1 sized capsules and 1000 - 1200 mg caplets.

| SUPPLEMENT | DOSAGE |
|---|---|
| Glyco-1 | 2 capsules, 4x/day |
| Phyto-1 | 1 caplet, 4x/day |
| Glyco-1 with dioscorea complex | 1 caplet, 4x/day |
| PROFILE^{™} | 1 tablet, 3x/day |

As the exemplified studies proceeded, the doses of the supplements were modified according to patient response to a prior dosing regimen. For example, if a patient's overall health was not improving at the initial dose, the respective dose for one or more of the supplements was modified. It should be noted that the actual doses ultimately given to each patient in a study varied greatly from patient to patient as nutritional response varied. Generally, the dietary supplement and each of the other supplements was administered in the range of about 1 to about 12 capsules (or caplets or tablets) per day.

It is well documented that biochemical individuality exists among mammals and results in a very wide range of drug or food required to obtain a desirable health promoting effect. (Williams, R.; in Nutrition Against Disease, 1971). The amount of the above nutraceuticals typically utilized initially as a dietary supplement is indicated for conditions of compromised health. Energy level, stiffness, pain, discomfort, restful sleep, recovery from fatigue, and emotional status are used as nutritional benefit markers in determining a mammal's nutritional response to the dietary supplement and in determining whether or not an increase in dose is warranted. A reduction of health complaints or a reduction of elimination of the above parameters is used as a guide for the reduction of glyconutrient intake. Complicating factors in regard to the amount of glyconutrients required for a benefit include the differing quantitative needs that individual have for nutrients, the differences being due to genetics, biochemical balance, disease state, altered physiology, prior and current general nutrition, individual choice and the nutrient content of food eaten by individuals. A desirable response or improvement in health is obtained when the missing nutrient or nutrients is/are adequately supplied by the present dietary supplement. The human body defends, repairs, regenerates, regulates, and heals itself through gene-control and nutrition provides the resources to accomplish these tasks. The inventive dietary supplements herein contain glyconutrients no longer commonly found in the urban/suburban food chain and thus supply a more optimal source of known and yet to be identified nutrients necessary for optimal biochemistry and physiology.

### EXAMPLE 1

A suitable composition for a product according to the present invention is as follows: 25 kilograms each of galactose, glucose, mannose, N-acetylneuraminic acid, fucose, N-acetylgalactosamine, N-acetylglucosamine, and xylose available from Florida Food Products as well as Aldrich Chemical Company and Sigma Chemical is charged into a stainless steel ribbon blender and mixed for five (5) minutes. Then 250 grams of Aerosil 380™ (silica gel) is added to the mixture as a flowing agent and 200 kilograms of rice flour, a source of glucose, is added as a gluten-free filler. The mixture is then agitated for fifteen (15) minutes. Finally, 100 grams of calcium stearate is added to the mixture as a lubricant and the mixture is agitated for an additional three (3) minutes to generate a bulk powder. The powder is then encapsulated into size #1 gelatin capsules at a fill weight of 250 mg using a Model H (Elanco) capsule filling machine.

### EXAMPLE 2

### Standardization Assay

The following assay describes a method for standardization of concentrations of sugars covered by this patent.
*Standards:* All carbohydrate standards are available from Aldrich Chemical Company, Milwaukee, Wisconsin.
*Eluent:* Deionized (DI) water having a resistance greater than or equal to about 17Mohm.
*Sample preparation:* 2 ml of 2 N hydrofluoric acid are added to 10 mg of sample to be analysed in a screw-top, TEFLON lined 10 ml test tube. The sample is then incubated at 120° C for one hour to hydrolyse into monosaccharides. The excess reagent is removed under a stream of air and the sample is resuspended in 1 ml of DI water.
*HPLC analysis:* AOAC Official Methods of Analysis 977.20.

In summary, this invention pertains to the field of dietary supplements and nutritional support for promotion and maintenance of good health. More specifically, the invention relates to compositions of carbohydrates as dietary supplements that are essential for the production of correctly structured and, therefore, properly functioning glycoproteins.

Science has recently shown that glycoproteins play a key role in all cellular communication. Many of the cytokines, i.e. cellular "words," do not function properly without an attached glycosyl moiety. The body hydrolyzes complex polysaccharides such as plant carbohydrates into various monosugars and restructures them into oligosaccharides that are then used by the body to build the glycoproteins required by cytokines for cellular communication and, thus, for good health.

This invention will correct the problem caused by modern diets consisting of highly refined foods, from which many essential ingredients have been eliminated during processing, specifically sugars needed for correctly structured and properly functioning gycoproteins.

The above is a detailed description of particular embodiments of the invention. Those of skill in the art should, in light of the present disclosure, appreciate that obvious modifications of the embodiments disclosed herein can be made without departing from the scope of the invention. All of the embodiments disclosed herein can be made and executed without undue experimentation in light of the present disclosure.

As used herein and unless otherwise indicated, the terms "a" and "an" are taken to mean "one", "at least one" or "one or more".

## Claims

1. A dietary supplement for providing nutritional product saccharides, which saccharides are essential components of glycoproteins in a mammal, said dietary supplement comprising nutritionally effective amounts of:
at least six monosaccharides selected from the group consisting of:
galactose, glucose, mannose, xylose, acetylated mannose, N-acetylneuraminic acid, fucose, N-acetylga lactosamine, N-acetylglucosamine, arabinose, glucuronic acid, galacturonic acid, iduronic acid, glucosamine and galactosamine.

2. A dietary supplement according to claim 1, comprising:
galactose, glucose, mannose, N-acetylneuraminic acid, fucose, N-acetylgalactosamine, N-acetylglucosamine and xylose.

3. A dietary supplement according to claim 2, further comprising rice flour.

4. A dietary supplement according to claim 2, further comprising silica gel as a flowing agent and a lubricant.

5. A dietary supplement according to claim 3, wherein said galactose, glucose, mannose, N-acetylneuraminic acid, fucose, N-acetylgalactosamine, N-bcetylglucogainine, xylose and rice flour are present in a weight ratio of 1:1:1:1: 1:1:1:1:8. ' .

6. A dietary supplement according to any on of claims 1 to 5, further comprising a nutritionally effective amount of one or more of ripened and freeze-dried fruits and vegetables, dioscorea complex, herbal extracts, herbal body-toning agents, beta sitosterol, melatonin, and vitamins and minerals.

7. A dietary supplement according to claim 6, wherein said ripened and freeze-dried fruits and vegetables comprise one or more of broccoli, brussels sprouts, cabbage, carrot, cauliflower, garlic, kale, onion, papaya, pineapple, tomato and turnip.

8. A dietary supplement according to claim 7, comprising from 0.01 to 99.999 weight percent of said monosaccharides and from 0.001 to 99.99 weight percent of said ripened and freeze-dried fruits and vegetables.

9. A dietary supplement according to claim 7, comprising from to 80 weight percent of said monosaccharides and from 20 to 99 weight percent of said ripened and freeze-dried fruits and vegetables.

10. A dietary supplement according to claim 7, comprising from 5 to 50 weight percent of said monosaccharides and from 50 to 95 weight percent of said ripened and freeze-dried fruits and vegetables.

11. A dietary supplement according to claim 6, comprising from 50 to 99.9999 weight percent of said monosaccharides and from 0.0001 to 50 weight percent of said dioscorea complex.

12. A dietary supplement according to claim 6, comprising from 30 to 99.99 weight percent of said monosaccharides and from 0.01 to 70 weight percent of said dioscorea complex.

13. A dietary supplement according to claim 6, comprising from 60 to 90 weight percent of said monosaccharides and from 10 to 40 weight percent of said dioscorea complex.

14. A dietary supplement according to claim 6, comprising about 80 weight percent of said monosaccharides and about 20 weight percent of said dioscorea complex.

15. A dietary supplement according to claim 6, wherein said herbal extracts are extracted from, one or more of broccoli, brussels sprouts, cabbage, carrot, cauliflower, garlic, kale, onion, papaya, pineapple, tomato, asparagus, mushroom, parsnip, radish and turnip.

16. A dietary supplement according to claim 15, comprising from 25 to 99.999 weight percent of said monosaccharides and from 0.001 to 75 weight percent of said herbal extracts.

17. A dietary supplement according to claim 15, comprising from 10 to 90 weight percent of said monosaccharides and from 10 to 90 weight percent of said herbal extracts.

18. A dietary supplement according to claims 6, wherein:
said vitamins comprise A, B1, B12, B2, B6, beta carotene, bioflavonoids, biotin, C, cholihe, D, E, folic acid, inositol, K, niacinamide, para-aminobenzoic acid, and pantothenic acid; and
said minerals comprise boron, calcium, copper, GTF chromium,iodine, iron, magnesium, manganese, molybdenum, potassium, selenium, silicon, vanadium and zinc.

19. Use of nutritional product monosaccharides, in the manufacture of a dietary supplement, in accordance with any one of claims 1 to 18, for the promotion and maintenance of good health.

## Patentansprüche

1. Nahrungsergänzungsmittel zur Bereitstellung von Nahrungsproduktsacchariden, wobei diese Saccharide essentielle Komponenten von Glycoproteinen bei einem Säuger sind, wobei die Dosierungsform nutritiv wirksame Mengen von:
wenigstens sechs Monosacchariden umfasst, die aus der Gruppe bestehend aus Galactose, Glucose, Mannose, Xylose, acetylierter Mannose, N-Acetylneutraminsäure, Fucose, N-Acetylgalactosamin, N-Acetylglucosamin, Arabinose, Glucuronsäure, Galacturonsäure, Iduronsäure, Glucosamin und Galactosamin ausgewählt sind.

2. Nahrungsergänzungsmittel nach Anspruch 1, das Galactose, Glucose, Mannose, N-Acetylneuraminsäure, Fucose, N-Acetylgalactosamin, N-Acetylglucosamin und Xylose umfasst.

3. Nahrungsergänzungsmittel nach Anspruch 2, das außerdem Reismehl umfasst.

4. Nahrungsergänzungsmittel nach Anspruch 2, das außerdem Silicagel als Fließmittel und Gleitmittel umfasst.

5. Nahrungsergänzungsmittel nach Anspruch 3, wobei Galactose, Glucose, Mannose, N-Acetylneuraminsäure, Fucose, N-Acetylgalactosamin, N-Acetylglucosamin, Xylose und Reismehl in einem Gewichtsverhältnis von 1:1:1:1:1:1:1:1:8 vorliegen.

6. Nahrungsergänzungsmittel nach einem der Ansprüche 1 bis 5, das außerdem eine nutritiv wirksame Menge eines oder mehrerer von gereiften und gefriergetrockneten Früchten und Gemüsen, Dioscorea-Komplex, Kräuterextrakten, Kräuter-Body-Toning-Mitteln, β-Sitosterol, Melatonin und Vitaminen und Mineralstoffen umfasst.

7. Nahrungsergänzungsmittel nach Anspruch 6, wobei die gereiften und gefriergetrockneten Früchte und Gemüse eins oder mehrere von Broccoli, Rosenkohl, Kohl, Karotten, Blumenkohl, Knoblauch, Grünkohl, Zwiebel, Papaya, Ananas, Tomate und Steckrübe sind.

8. Nahrungsergänzungsmittel nach Anspruch 7, das 0,01 bis 99,999 Gew.-% der Monosaccharide und 0,001 bis 99,99 Gew.-% der gereiften und gefriergetrockneten Früchte und Gemüse umfasst.

9. Nahrungsergänzungsmittel nach Anspruch 7, das 1 bis 80 Gew.-% der Monosaccharide und 20 bis 99 Gew.-% der gereiften und gefriergetrockneten Früchte und Gemüse umfasst.

10. Nahrungsergänzungsmittel nach Anspruch 7, das 5 bis 50 Gew.-% der Monosaccharide und 50 bis 95 Gew.-% der gereiften und gefriergetrockneten Früchte und Gemüse umfasst.

11. Nahrungsergänzungsmittel nach Anspruch 6, das 50 bis 99,9999 Gew.-% der Monosaccharide und 0,0001 bis 50 Gew.-% des Dioscorea-Komplexes umfasst.

12. Nahrungsergänzungsmittel nach Anspruch 6, das 30 bis 99,99 Gew.-% der Monosaccharide und 0,01 bis 70 Gew.-% des Dioscorea-Komplexes umfasst.

13. Nahrungsergänzungsmittel nach Anspruch 6, das 60 bis 90 Gew.-% der Monosaccharide und 10 bis 40 Gew.-% des Dioscorea-Komplexes umfasst.

14. Nahrungsergänzungsmittel nach Anspruch 6, das etwa 80 Gew.-% der Monosaccharide und etwa 20 Gew.-% des Dioscorea-Komplexes umfasst.

15. Nahrungsergänzungsmittel nach Anspruch 6, wobei die Kräuterextrakte aus einem oder mehreren von Broccoli, Rosenkohl, Kohl, Karotten, Blumenkohl, Knoblauch, Grünkohl, Zwiebel, Papaya, Ananas, Tomate, Spargel, Pilz, Pastinake, Radieschen und Steckrübe extrahiert sind.

16. Nahrungsergänzungsmittel nach Anspruch 15, das 25 bis 99,999 Gew.-% der Monosaccharide und 0,001 bis 75 Gew.-% der Kräuterextrakte umfasst.

17. Nahrungsergänzungsmittel nach Anspruch 15, das 10 bis 90 Gew.-% der Monosaccharide und 10 bis 90 Gew.-% der Kräuterextrakte umfasst.

18. Nahrungsergänzungsmittel nach Anspruch 6, wobei:
die Vitamine A, B1, B12, B2, B6, β-Carotin, Bioflavonoide, Biotin, C, Cholin, D, E, Folsäure, Inositol, K, Niacinamid, para-Aminobenzoesäure und Pantothensäure umfassen; und
die Mineralstoffe Bor, Calcium, Kupfer, GTF-Chrom, Iod, Eisen, Magnesium, Mangan, Molybdän, Kalium, Selen, Silicium, Vanadium und Zink umfassen.

19. Verwendung von Nahrungsproduktmonosacchariden in der Herstellung eines Nahrungsergänzungsmittels nach einem der Ansprüche 1 bis 18 zur Förderung und Aufrechterhaltung einer guten Gesundheit.

## Revendications

1. Supplément diététique pour fournir des saccharides de produit nutritionnel, lesquels saccharides sont des composants essentiels de glycoprotéines chez un mammifère, ledit supplément diététique comprenant des quantités nutritionnellement efficaces :
d'au moins six monosaccharides choisis dans le groupe constitué par : galactose, glucose, mannose, xylose, mannose acétylé, acide N-acétylneuraminique, fucose, N-acétylgalactosamine, N-acétylglucosamine, arabinose, acide glucuronique, acide galacturonique, acide iduronique, glucosamine et galactosamine.

2. Supplément diététique selon la revendication 1, comprenant :
galactose, glucose, mannose, acide N-acétylneuraminique, fucose, N-acétylgalactosamine, N-acétylglucosamine et xylose.

3. Supplément diététique selon la revendication 2, comprenant en outre de la farine de riz.

4. Supplément diététique selon la revendication 2, comprenant en outre du gel de silice comme agent d'écoulement et un lubrifiant.

5. Supplément diététique selon la revendication 3, dans lequel lesdits galactose, glucose, mannose, acide N-acétylneuraminique, fucose, N-acétylgalactosamine, N-acétylglucosamine, xylose et farine de riz sont présents en un rapport en poids de 1 : 1 : 1 : 1 : 1 : 1 : 1 : 1 : 8.

6. Supplément diététique selon l'une quelconque des revendications 1 à 5, comprenant en outre une quantité nutritionnellement efficace d'un ou plusieurs éléments parmi des fruits et légumes à maturité et lyophilisés, un complexe de dioscorea, des extraits d'herbes aromatiques, des agents à base d'herbes pour tonifier le corps, le bêta sitostérol, la mélatonine et des vitamines et minéraux.

7. Supplément diététique selon la revendication 6, dans lequel lesdits fruits et légumes à maturité et lyophilisés comprennent un ou plusieurs fruits et légumes parmi les brocolis, les choux de Bruxelles, le chou, la carotte, le chou-fleur, l'ail, le chou vert frisé, l'oignon, la papaye, l'ananas, la tomate et le rutabaga.

8. Supplément diététique selon la revendication 7, comprenant de 0,01 à 99,999 pour cent en poids desdits monosaccharides et de 0,001 à 99,99 pour cent en poids desdits fruits et légumes à maturité et lyophilisés.

9. Supplément diététique selon la revendication 7, comprenant 80 pour cent en poids desdits monosaccharides et de 20 à 99 pour cent en poids desdits fruits et légumes à maturité et lyophilisés.

10. Supplément diététique selon la revendication 7, comprenant de 5 à 50 pour cent en poids desdits monosaccharides et de 50 à 95 pour cent en poids desdits fruits et légumes à maturité et lyophilisés.

11. Supplément diététique selon la revendication 6, comprenant de 50 à 99,9999 pour cent en poids desdits monosaccharides et de 0,0001 à 50 pour cent en poids dudit complexe de dioscorea.

12. Supplément diététique selon la revendication 6, comprenant de 30 à 99,99 pour cent en poids desdits monosaccharides et de 0,01 à 70 pour cent en poids dudit complexe de dioscorea.

13. Supplément diététique selon la revendication 6, comprenant de 60 à 90 pour cent en poids desdits monosaccharides et de 10 à 40 pour cent en poids dudit complexe de dioscorea.

14. Supplément diététique selon la revendication 6, comprenant environ 80 pour cent en poids desdits monosaccharides et environ 20 pour cent en poids dudit complexe de dioscorea.

15. Supplément diététique selon la revendication 6, dans lequel lesdits extraits d'herbes aromatiques sont extraits de un ou plusieurs fruits et légumes parmi les brocolis, les choux de Bruxelles, le chou, la carotte, le chou-fleur, l'ail, le chou vert frisé, l'oignon, la papaye, l'ananas, la tomate, l'asperge, le champignon, le panais, le radis et le rutabaga.

16. Supplément diététique selon la revendication 15, comprenant de 25 à 99,999 pour cent en poids desdits monosaccharides et de 0,001 à 75 pour cent en poids desdits extraits d'herbes aromatiques.

17. Supplément diététique selon la revendication 15, comprenant de 10 à 90 pour cent en poids desdits monosaccharides et de 10 à 90 pour cent en poids desdits extraits d'herbes aromatiques.

18. Supplément diététique selon la revendication 6, dans lequel :
lesdites vitamines comprennent A, B1, B12, B2, B6, bêta carotène, bioflavonoïdes, biotine, C, choline, D, E, acide folique, inositol, K, niacinamide, acide para-aminobenzoïque et acide pantothénique ; et lesdits minéraux comprennent le bore, le calcium, le cuivre, le chrome à facteur de tolérance au glucose, l'iode, le fer, le magnésium, le manganèse, le molybdène, le potassium, le sélénium, le silicium, le vanadium et le zinc.

19. Utilisation de monosaccharides de produit nutritionnel, dans la fabrication d'un supplémentaire diététique, conformément à l'une quelconque des revendications 1 à 18, pour le développement et l'entretien d'une bonne santé.
